# EUROPEAN PATENT SPECIFICATION

(11) **EP 0 165 970 B1**
(45) Date of publication and mention of the grant of the patent: **03.03.1993**
(21) Application number: 85900364.2
(22) Date of filing: 30.11.1984
(51) Int. Cl.: A61K 31/56

(54) **HAIR GROWTH MODIFICATION**
HAARWUCHSMODIFIKATION
MODIFICATION DE LA CROISSANCE PILAIRE

(30) Priority: 12.12.1983 US 560726
(43) Date of publication of application: 02.01.1986
(73) Proprietor: KASZYNSKI, Edwin G., Wheaton, MD 20902 (US)
(72) Inventor: BREUER, Miklos, M., Newton, MA 02159 (US); SHANDER, Douglas, Gaithersburg, MD 20878 (US); USDIN, Vera, R., Rockville, MD 20850 (US); VAN DER LEE, Hermes, Silver Spring, MD 20906 (US)
(74) Representative: Baillie, Iain Cameron
(86) International application number: US8401977
(87) International publication number: WO8502543

(56) References cited:
- DE-A- 2 840 144
- US-A- 4 039 669
- US-A- 4 098 802
- US-A- 4 269 831
- US-A- 4 310 523
- US-A- 4 439 432

## Description

This invention relates to a cosmetic process for reducing the rate and altering the character of androgen-stimulated beard hair growth in intact, sexually mature males, which comprises applying to the skin a composition comprising at least one antiandrogen agent consisting of a 5-alpha-reductase inhibitor or a cytoplasmic androgen receptor-binding agent or a mixture thereof, and a dermatologically acceptable carrier.

A great deal of medical investigation has been directed to the elucidation of the role of the endocrine system in the growth of human hair. As the result of such investigations, it is generally agreed that the fine, light colored vellus hair, which covers most of the body during childhood, comes under the influence of growth hormone and of androgens to eventually become the coarser and darker terminal hairs which characterize many areas of the adult body.

In each body area, the character of the final hair growth is determined not only by the level of circulating androgen and the sensitivity of the follicles in that area to androgen, but also to the length of time over which the follicles have been exposed. Both pubic and axillary hair appear early in puberty but decrease with the onset of the androgen deficiency of old age. Beard growth and balding, on the other hand, develop late in puberty and outlast the natural diminution of androgen. The desire to discover methods for controlling androgen-dependent conditions has generated a large number of studies dealing with androgen metabolism in skin.

As a result of these studies, it is thought that all steroids, including testosterone, the principal androgen circulating in the plasma in men, enter target tissues by passive diffusion. In some tissues, such as muscle, testosterone binds directly to a cytoplasmic receptor protein permitting translocation of the hormone into the cell mucleus. Once inside, the testosterone receptor complex beings about synthesis of new proteins necessary for expression of virilizing activities by the cell.

In other tissues, such as skin, intracellular testosterone is first reduced by the enzyme steroid 5-alpha-reductase to the compound dihydrotestosterone before association with the receptor and subsequent nuclear translocation takes place. Thus, in the treatment of androgen-mediated conditions in skin, it is possible to reduce the amount of androgen capable of entering the nucleus by two means.

Firstly, the conversion of serum testosterone to dihydrotestosterone can be prevented by the inhibition of the enzyme steroid 5-alpha-reductase. Secondly, certain compounds can compete with the testosterone or dihydrotestosterone for the cytoplasmic receptor sites. Our invention is the first to demonstrate altering beard hair growth by topical application of antiandrogens (5-alpha-reductase inhibitors and/or cytoplasmic receptor-binding agents) in adult males. The action of such antiandrogen compounds in skin can also affect the output of sebaceous glands and the course of male pattern hair growth in females, thus leading to their application in the treatment of acne and female hirsutism. The ideal antiandrogenic agent for purposes of altering beard hair growth is one which is active topically with no significant side effects. Although several topically active antiandrogens have been reported in the art, none claim or teach an effect on male beard hair growth.

U.S. Patent US-A-4,039,669 describes the topical use of 17-alpha-R-androst-4-en-17-beta-ol 3-one or esters thereof where the R is n-propyl or n-butyl for the control of dermatological conditions associated with androgen-mediated conditions such as acne.

U.S. Patents US-A-4,139,638 and US-A-4,151,540 describe the use of certain 4'-substituted and 3',4'-disubstituted anilides for the treatment of androgen-dependent disease states such as female hirsutism and acne.

U.S. Patent US-A-4,191,775 discloses that certain 3,4-disubstituted-branched-chain-fluorinated-acylanilides may be used in the topical treatment of androgen-dependent disease conditions such as acne, female hirsutism, and seborrhoea.

U.S. Patent US-A-4,344,941 describes the topical use of certain androgenic 17-alpha-substituted steroids exemplified by 17-beta-hydroxy-1-alpha-methyl-17-alpha-(1-methyl-2-propenyl)-5-alpha-androstan-3-one for the treatment of diseases such as acne, seborrhoea, alopecia and female hirsutism.

U.S. Patent US-A-4,367,227 describes a cosmetic composition for reducing sebum secretion from the skin comprising alcoholic solutions of cyproterone acetate.

West German OLS DE-B-2,840,144 describes the use of combinations of progesterone with either cyproterone acetate or chlormadinone acetate in the topical treatment of androgen-induced hormonal disturbances such as alopecia, female hirsutism, and acne.

Japanese Kokai JP-A-58-57308 describes the restoration of hair to bald heads by the topical application of oxidizing substances such as stabilized chlorine dioxide, potassium bromate, or ozone to supress the enzymatic activity of the reductive enzyme 5-alpha-reductase.

The patent art discloses a number of ways of reducing the growth of human hair as opposed to its conventional removal by cutting, shaving or depilation. One such method is described in U.S. Patent US-A-3,426,137 which pertains to a process for inhibiting the growth of hair by the topical application to a depilated skin area of a composition containing a substituted benzophenone such as 2-amino-5-chlorobenzophenone. Examples in the patent illustrate the reduction of hair growth on the back area of rabbits and on the arm of a male human subject.

Another process for extending the duration of depilation is described in U.S. Patent US-A-4,370,315. The process therein comprises the topical application of a composition containing a lipoxygenase along with linoleic acid or derivative thereof. The patent describes the application of such composition to various body parts of female human subjects in the majority of which regrowth of hair was clearly perceptible only after six or more weeks.

We have discovered that antiandrogen materials of two classes, namely steroid 5-alpha-reductase inhibitors and cytoplasmic androgen receptor binding agents may be employed in altering the rate and character of androgen-stimulated hair growth in the beards of intact, sexually mature males. The antiandrogens are applied topically out of a dermatologically acceptable carrier for local effect and with minimal alteration of other androgen-mediated bodily functions through systemic action. In a preferred practice of the invention, compositions containing both a steroid 5-alpha-reductase inhibitor along with a cytoplasmic androgen receptor binding agent are employed.

We have discovered that the normal rate of male beard hair growth can be reduced and its character caused to revert toward the vellus state by the topical application of anti-androgen compounds of either the 5-alpha-reductase inhibitor type or the cytoplasmic androgen receptor binding agent type. By the proper selection of antiandrogen compound and its mode of use, unwanted interference with other androgen-mediated bodily processes can be minimized or avoided.

The absence of antagonism of synthemic androgen activity in the practice of our invention can be shown by the topical application of suitable compositions to one of the two flank organs of mature male hamsters. Dose-related decreases in androgen-dependent flank organ hair growth are obtained on the treated side but no statistically significant change occurs in the contralateral, vehicle-treated side. Similar studies have been conducted using female hamsters in which hair growth on the flank organs has been stimulated by the subcutaneous administration of a suitable androgen.

A novel method for screening topical antiandrogens has been developed in which the fluctuations in flank organ activity of the enzyme ornithine decarboxylase (ODC) in response to androgens and antiandrogens is exploited to provide a rapid, in vivo screen for antiandrogens, based on a biochemical parameter specifically related to androgenic stimulation of pilosebaceous target tissues. In several studies, unilateral topical application of the antiandrogens 17-alpha-propyltestosterone and 17-alpha-allyltestosterone selectively decreased ODC activity on treated but not untreated flank organs of intact (i.e. uncastrated) male hamsters. Treatment of androgen stimulated female hamsters with topical cyproterone acetate or progesterone also inhibited ODC activity.

A further series of investigations on the androgenic regulation of ODC activity in hamster flank organs has been conducted to test topical antiandrogenic formulations consisting of combination regimens. Anti-androgens, both androgen-binding inhibitors (examples being cyproterone acetate, chlormadinone acetate, 17-alpha-propyl-testosterone, and 17-alpha-allyltestosterone) and 5-alpha-reductase inhibitors such as progesterone were found to inhibit ODC activity in flank organs when applied topically. However, the combination of progesterone with binding inhibitors enhanced the magnitude of ODC inhibition and allowed a reduction of the dose of binding inhibitors necessary for maximal local efficacy. Combination formulations containing low doses of androgen-binding inhibitors appeared to be the most effective topical antiandrogens producing high local and low contralateral effects. Combinations containing progesterone and 17-alpha-propyltestosterone or 17-alpha-allyl-testosterone provided maximal local inhibition and, in contrast to other regimens, did not cause any contralateral effects.

Summarized below are the results of several experiments comparing the efficacy of several compositions in reducing the flank organ hair mass in adult intact male hamsters. In each case, hamsters were treated for 15 days (Monday-Friday) during a 21-day interval. Flank organ hairs were depilated on the first day of treatment and redepilated on the sixth day of treatment. The mass of flank organ hair represents the re-growth during the final 14 days of the 21-day interval. The results for percent inhibition shown below are based upon comparisons between the hair mass values of hairs hervested from treated flank organs of experimental animals and those obtained from vehicle-treated control animals.

| Experiment I | | |
|---|---|---|
| Steroidal Antiandrogen(s) | Rate of Application in Micrograms per Square Centimeter | % Inhibition |
| Progesterone | 1000 | 30 |
| Cyproterone acetate | 500 | 26 |
| 17-α-Propyltestosterone | 6 | 26 |
| 17-α-Propyltestosterone | 6 | |
| + Progesterone | 1000 | 39 |
| Cyproterone acetate | 500 | |
| + Progesterone | 1000 | 52 |

| Experiment II | | |
|---|---|---|
| Steroidal Antiandrogen(s) | Rate of Application in Micrograms per Square Centimeter | % Inhibition |
| Cyproterone acetate | 500 | 38 |
| Cyproterone acetate | 50 | 23 |
| 4- androstene- 3-one -17B- Carboxylic acid | 400 | 29 |
| Cyproterone acetate | 50 | |
| + 4- androstene- 3-one -17B- Carboxylic acid | 400 | 41 |
| Cyproterone acetate | 500 | |
| + 4- androstene- 3-one -17B- Carboxylic acid | 400 | 54 |

| Experiment III | | |
|---|---|---|
| Steroidal Antiandrogen(s) | Rate of Application in Micrograms per Square Centimeter | % Inhibition |
| Progesterone | 1000 | 57 |
| Spironolactone | 500 | 41 |
| Chlormadione acetate | 500 | 45 |
| Chlormadione acetate | 500 | |
| + Progesterone | 1000 | 69 |

| Experiment IV | | |
|---|---|---|
| Steroidal Antiandrogen(s) | Rate of Application in Micrograms per Square Centimeter | % Inhibition |
| Progesterone | 1000 | 48 |
| 17-α-allyltestosterone | 5 | 0 |
| 17-α-allyltestosterone | 20 | 29 |
| 17-α-allyltestosterone | 50 | 50 |
| 17-α-allyltestosterone | 100 | 50 |

In employing the topical application of antiandrogens in altering the rate and character of beard hair growth, one may use a variety of 5-alpha-reductase inhibitors and cytoplasmic receptor binding agents either alone or in combination with each other. Among the 5-alpha-reductase inhibitors that may be employed are progesterone; (5α,20-R)-4-diazo-21-hydroxy-20-methyl pregnan-3-one; (4R)-5-10-seco-19-Norpregna4,-5-diene-3,10,20 trione; 4-androstene-3-one-17β-carboxylic acid, and its methyl ester; 17-β-N,N-diethylcarbamoyl-9-methyl4-aza-5α-androstane-3-one; 11-α-OH-progesterone; 17-α-OH-progesterone; and 20-α-OH-progesterone. For minimum alteration of other androgen-mediated bodily functions through systemic action, we prefer to use progesterone or 4-androstene-3-one-17 carboxylic acid. The concentration and level of application of these materials in formulated compositions as discussed below should be such that from 10 to 10,000 µg of active material per square centimeter of skin will be applied. We prefer compositions which will result in the application of 100 to 1,000 µg per square centimeter.

Among the cytoplasmic receptor binding agents, which may be mentioned, are cyproterone acetate, chlormadinone acetate, 17-alpha-propyltestosterone, 17-alpha-allyltestosterone, α-α-α-trifluoro-2- methyl-4'-nitro-m-propionotoluidide; 6α-bromo-17β-hydroxy-17α-methyl-4-oxa-5α-androstane-3-one; 17β-acetoxy-4α,5cyclo-A-homo-B-nor-5α-androst-1-ene-3one; and spironolactone. For minimal alteration of other androgen-mediated bodily functions through systemic action we prefer to use 17-alpha-propyltestosterone or 17-alpha-allyltestosterone. The concentration and level of application of these materials in formulated compositions as discussed below should be such that from 0.1 to 5,000 µg of active material per square centimeter of skin will be applied. We prefer compositions which result in the application of 1.0 to 500 µg per square centimeter.

We prefer to employ 5-alpha-reductase inhibitors and cytoplasmic androgen receptor binding agents in combination with each other because the 5-alpha-reductase inhibitors appear to enhance the action of the cytoplasmic androgen receptor binding agents, permitting a lower concentration to be used thus reducing the risk of systemic side effects. When used in combination, the 5-alpha-reductase inhibitors and cytoplasmic androgen receptor binding agents should be employed within the ranges of application discussed above for separate use of the 5-alpha-reductase inhibitors and cytoplasmic androgen receptor binding agents.

In formulating the compositions to be applied topically in the practice of this invention, any dermatologically acceptable base or carrier may be employed. Care should be taken, however, to use a base or carrier which will provide uniform localized absorption of the antiandrogen principle without significant systemic absorption. The art practiced in the formulation of skin creams for cosmetic purposes may usefully be employed in the formulation of compositions used in the practice of our invention. For example, many derivatives of lanolin are known to have excellent emulsifying properties and may be used to facilitate the formulation of emulsions having critical stability requirements. Lanolin has also been thought to aid in the absorption of active materials into the skin. While the active materials may be incorporated in a variety of cosmetic-based materials such as simple solutions, creams, suspensions, gels and the like, water-in-oil type cream emulsions may offer advantages in that the continuous oil provides direct contact with the lipids of the skin to provide a route for slow continuous absorption of the active antiandrogen.

In formulating compositions containing 5-alpha-reductase inhibitors, it is possible to include as little as 0.1% or as much as 10.0% by weight in the practice of our invention. We prefer to use from 1.0 to 4.0% by weight. In formulating compositions containing cytoplasmic receptor binding agents, it is likewise possible to use from 0.1 to 10.0% by weight. We prefer, however, to use from 0.5 to 5.0% by weight in the case cyproterone acetate, chlormadinone acetate, and spironolactone and from 0.2 to 0.5% by weight in the case of other cytoplasmic receptor binding agents.

In using the antiandrogen-containing compositions described herein in altering human beard hair growth, sufficient quantity of the composition is rubbed into the bearded area of the skin of the face and neck preferably on a daily basis to provide the level of application discussed above. Continued use will result in a reduction of beard hair mass and a gradual reversion toward the vellus state. The maximum rate of change which will be achieved will vary from individual to individual.

The following examples are illustrative of compositions to be used in the practice of the invention.

| Example 1 - Skin Lotion | |
|---|---|
| Ingredients | Weight % |
| Progesterone | 2.2 |
| Cetyl alcohol | 4.0 |
| Mineral Oil | 4.0 |
| Isopropyl Myristate | 1.0 |
| Dimethicone | 1.0 |
| Lanolin Alcohol | 0.5 |
| Glycerol monosterate | 1.0 |
| Sodium lactate (60% aq. soln.) | 1.4 |
| Dimethyl diammonium chloride (75% active)-Arquad 2HT75 | 2.0 |
| Propylene glycol | 3.0 |
| Methyl paraben | 0.2 |
| Propyl paraben | 0.1 |
| Titanium dioxide | 0:1 |
| Perfume | 0.1 |
| Water | 77.4 |
| | 1̅0̅0̅.̅0̅ |

Procedure: Deionized water and propylene glycol are heated to 70°C. Methyl paraben is added under high sheer agitation. In another container combine emollient oils, emulsifier, pre-warmed dimethyl diammonium chloride, active ingredients and propyl paraben. Heat and maintain 70°C. with moderate agitation for 30 minutes. Add the water phase to the oil phase and agitate moderately. Add titanium dioxide and mix for 60 minutes. Cool batch slowly to 55°C., add the sodium lactate (60%) and continue to cool slowly with agitation to room temperature.

| Example 2 - Cream Emulsion | | |
|---|---|---|
| Concentrate | | % w/w |
| A. | Cyproterone acetate | 2.2 |
| | Stearate acid xxx | 7.6 |
| | Amerchol L-101 | 5.0 |
| | Modulon | 2.0 |
| | Cetyl alcohol | 3.0 |
| | Propyl Parasept | 0.1 |
| B. | Glycerin | 4.0 |
| | Methyl Paraben | 0.15 |
| | Water | 75.95 |

Procedure: Combine (A) ingredients and heat to 70°C. Combine (B) ingredients separately and heat to 72°C. Add (B) to (A) with rapid stirring, then cool to room temperature.

| Example 3 - Aerosol Spray | | |
|---|---|---|
| Concentrate | | % w/w |
| A. | Magnesium aluminum silicate (Veegum K) | 1.5 |
| | Propylene glycol | 3.0 |
| | Water | 86.0 |
| B. | Chlormadinone acetate Diethylene glycol monostearate s.e. | 3.0 |
| | Silicone 556 Fluid | 1.0 |
| | Cetyl alcohol | 0.5 |
| | Acetylated lanolin alcohols | 2.0 |
| | Preservative | 0.2 |

Procedure: Add the Veegum to water slowly with rapid agitation, until smooth. Add remaining (A) ingredients and heat to 80°C. Combine (B) ingredients and heat to 75°C. Add (A) to (B) with mixing and cool to room temperature. Package as an aerosol by combining 90 parts of concentrate with 10 parts of hydrocarbon propellent A-46.

| Example 4 - Aerosol Foam | | |
|---|---|---|
| Concentrate | | % w/w |
| A. | Progesterone | 2.0 |
| | Cyproterone acetate | 0.2 |
| | Cetyl alcohol | 5.2 |
| | Polyoxyethylene (401 stearate (MYRJ52)) | 3.0 |
| B. | Propylene glycol | 4.0 |
| | Water | 85.4 |
| | Preservative | 0.2 |

Procedure: Combine (A) ingredients and heat to 70°C. Combine (B) ingredients separately and heat to 72°C. Add (B) to (A) with mixing and cool to room temperature. Package as aerosol using a ratio of 7 parts hydrogen propellent A-31 to 93 parts of concentrate.

| Example 5 - Alcohol Solution | |
|---|---|
| Concentrate | % w/w |
| 17-alpha-propyltestosterone | 2.2 |
| Propylene glycol | 4.0 |
| Dimethicone | 1.0 |
| SDA-40 Alcohol | 92.8 |

Procedure: Combine ingredients with mixing and package.

## Claims

1. A cosmetic process for reducing the rate and altering the character of androgen-stimulated beard hair growth in intact, sexually mature males, which comprises applying to the skin a composition comprising at least one antiandrogen agent consisting of a 5-alpha-reductase inhibitor or a cytoplasmic androgen receptor-binding agent or a mixture thereof, and a dermatologically acceptable carrier.

2. A process according to claim 1, in which the 5-alpha-reductase inhibitor is progesterone; (5α,20-R)-4-diazo-21-hydroxy-20-methyl pregnan-3-one; (4R)-5-10-seco-19-Norpregna-4,5-diene-3,10,20 trione; 4-androstene-3-one-17β-carboxylic acid, or its methyl ester; 17-β-N,N-diethylcarbamoyl-9-methyl-4-aza-5α-androstane3-one; 11-α-OH-progesterone; or 20-α-OH-progesterone.

3. A process according to claim 2, wherein the concentration of said antiandrogen agent is selected to result in the application from 10 to 10,000 µg of 5-alpha-reductase inhibitor per square centimeter of skin.

4. A process according to claim 2, wherein the concentration of said antiandrogen agent is selected to result in the application of from 100 to 1,000 µg of 5-alpha-reductase inhibitor per square centimeter of skin.

5. A process according to claim 1, in which the cyctoplasmic androgen receptor-binding agent is cyproterone acetate, chlormadinone acetate, 17-alpha-propyltestosterone; 17-alpha-allyltestosterone; α-α-α-trifluoro-2-methyl-4'-nitro-m-propionotoluidide; 6α-bromo-17β-hydroxy-17α-methyl-4-oxa-5α-androstane-3-one; 17β-acetoxy-4α,5cyclo-A-homo-B-nor-5α-androst-1-ene-3one; or spironolactone.

6. A process according to claim 5, wherein the concentration of said antiandrogen agent is selected to result in the application of from 0.1 to 5,000 µg of the cytoplasmic androgen receptor-binding agent per square centimeter of skin.

7. A process according to claim 5, wherein the concentration of said antiandrogen agent is selected to result in the application from 1.0 to 500 µg of cyctoplasmic androgen receptor-binding agent per square centimeter of skin.

8. A process according to any one of the preceding claims, in which a 5-alpha-reductase inhibitor is used along with a cyctoplasmic androgen receptor-binding agent.

9. The cosmetic use of a composition as defined in any one of the preceding claims 1 to 10, to reduce the rate and alter the character of androgen-stimulated beard hair growth in intact, sexually mature males.

## Patentansprüche

1. Kosmetisches Verfahren zur Verminderung der Geschwindigkeit und Änderung des Charakters von androgen-stimuliertem Barthaarwuchs bei gesunden, geschlechtsreifen Männern, bei dem eine Zusammensetzung auf die Haut aufgebracht wird, die wenigstens ein antiandrogenes Mittel enthält, welches aus einem 5-α-Reduktase-Hemmstoff oder einem Mittel, welches einen Androgenrezeptor aus dem Zytoplasma bindet, oder einer Mischung derselben besteht, und einen dermatologisch verwendbaren Träger.

2. Verfahren nach Anspruch 1, bei dem der 5-α-Reduktase-Hemmstoff Progesteron ist; (5α,20-R)-4-diazo-21-hydroxy-20-methyl pregnan-3-on; (4R)-5-10-seco-19-Norpregna-4,5-dien3,10,20-trion; 4-Androsten-3-on-17β-carbonsäure, oder ihr Methylester; 17-β-N,N-diethylcarbamoyl-9-methyl-4-aza-5α-androstan-3-on; 11-α-OH-progesteron; oder 20-α-OH-progesteron.

3. Verfahren nach Anspruch 2, dadurch gekennzeichnet, daß die Konzentration des antiandrogenen Mittels so gewählt ist, daß 10 bis 10.000 µg 5-α-Reduktase-Hemmstoff pro Quadratzentimeter Haut aufgetragen werden.

4. Verfahren nach Anspruch 2, dadurch gekennzeichnet, daß die Konzentration des antiandrogenen Mittels so gewählt ist, daß 100 bis 1.000 µg 5-α-Reduktase-Hemmstoff pro Quadratzentimeter Haut aufgetragen werden.

5. Verfahren nach Anspruch 1, bei dem das einen Androgenrezeptor aus dem Cytoplasma bindende Mittel Cyproteronacetat, Chlormadinonacetat, 17-α-Propyltestosteron; 17-α-allyltestosteron; α-α-α-Trifluor-2-methyl-4'-nitro-m-propionotoluidid; 6α-Bromo-17β-hydroxy-17α-methyl-4-oxa-5α-androstan-3-on; 17β-acetoxy-4α,5cyclo-A-homo-B-nor-5α-androst-1-en-3-on; oder Spironolacton ist.

6. Verfahren nach Anspruch 5, dadurch gekennzeichnet, daß die Konzentration des antiandrogenen Mittels so gewählt ist, daß 0,1 bis 5.000 µg des den Androgenrezeptor aus dem Zytoplasma bindenden Mittels pro Quadratzentimeter Haut aufgetragen werden.

7. Verfahren nach Anspruch 5, dadurch gekennzeichnet, daß die Konzentration des antiandrogenen Mittels so gewählt ist, daß 1,0 bis 500 µg des den Androgenrezeptor aus dem Zytoplasma bindenden Mittels pro Quadratzentimeter Haut aufgetragen werden.

8. Verfahren nach einem der vorhergehenden Ansprüche, bei dem ein 5-α-Reduktase-Hemmstoff zusammen mit einem den Androgenrezeptor aus dem Zytoplasma bindenden Mittel verwendet wird.

9. Kosmetische Verwendung einer Zusammensetzung nach einem der vorhergehenden Ansprüche 1 bis 10 zur Verminderung der Geschwindigkeit und Änderung des Charakters von androgen-stimuliertem Barthaarwuchs bei gesunden, geschlechtsreifen Männern.

## Revendications

1. Procédé cosmétique pour réduire la vitesse et modifier le caractère de la croissance des poils de barbe stimulée par des androgènes chez les mâles intacts, sexuellement matures, qui comprend l'application sur la peau d'une composition compenant au moins un agent antiandrogène consistant en un inhibiteur de la 5-alpha-réductase ou en un agent de liaison aux récepteurs d'androgènes cytoplasmiques ou en un mélange de ceux-ci, et un véhicule dermatologiquement acceptable.

2. Procédé selon la revendication 1, dans lequel l'inhibiteur de la 5-alpha-réductase est la progestérone ; la (5α,20-R)-4-diazo-21-hydroxy-20-méthyl prégnagne-3-one ; la (4R)-5-10-seco-19-Norpregna-4,5-diène-3,10,20 trione ; l'acide 4-androstène-3-one-17β-carboxylique ou son ester méthylique; la 17-β-N,N-diéthylcarbamoyl-9-méthyl-4-aza-5α-androstane-3-one ; la 11-α-OH-progestéronc ou la 20-α-OH-progestérone.

3. Procédé selon la revendication 2, dans lequel la concentration dudit agent antiandrogène est choisie de manière à permettre l'application de 10 à 10 000 µg d'inhibiteur de la 5-alpha-réductase par cm² de peau.

4. Procédé selon la revendication 2, dans lequel la concentration dudit agent antiandrogène est choisie de manière à permettre l'application de 100 à 1 000 µg d'inhibiteur de la 5-alpha-réductase par cm² de peau.

5. Procédé selon la revendication 1, dans lequel l'agent de liaison aux récepteurs d'androgènes cytoplasmiques est l'acétate de cyprotérone, l'acétate de chlormadinone, la 17-alpha-propyltestostérone, la 17-alpha-allyltestostérone, le α,α,α,trifluoro-2-méthyl-4'-nitro-m-propionotoluidide ; la 6α-bromo-17β-hydroxy--17α--méthyl-4--oxa-5α--androstane--3--one; la 17β--acétoxy-4α,5cyclo-A-homo-B-nor-5α-androst-1-ène-3one ou la spironolactone.

6. Procédé selon la revendication 5, dans lequel la concentration dudit agent antiandrogène est choisie pour permettre l'application de 0,1 à 5 000 µg d'agent de liaison aux récepteurs d'androgènes cytoplasmiques par cm² de peau.

7. Procédé selon la revendication 5, dans lequel la concentration dudit agent antiandrogène est choisie pour permettre l'application de 1,0 à 500 µg d'agent de liaison aux récepteurs d'androgènes cytoplasmiques par cm² de peau.

8. Procédé selon l'une quelconque des revendications précédentes, dans lequel un inhibiteur de la 5-alpha-réductase est utilisé avec un agent de liaison aux récepteurs d'androgènes cytoplasmiques.

9. Utilisation cosmétique des compositions selon l'une quelconque des revendications précédentes pour réduire la vitesse et modifier le caractère de la croissance des poils de barbe stimulée par des androgènes chez les mâles intacts, sexuellement matures.
